# FASCICULE DE BREVET EUROPEEN

(11) **EP 1 030 687 B1**
(45) Date de publication et mention de la délivrance du brevet: **24.08.2005**
(21) Numéro de dépôt: 99925113.5
(22) Date de dépôt: 16.06.1999
(51) Int. Cl.: A61K 47/06, A61K 47/02, A61K 9/16, A61K 9/06, A61K 7/00, A61K 7/48

(54) **COMPOSITION COSMETIQUE OU DERMO-PHARMACEUTIQUE SOUS FORME DE PERLES HYDROPHOBES ET PROCEDES POUR LA PREPARATION**
KOSMETISCHE ODER DERMO-PHARMAZEUTISCHE ZUSAMMENSETZUNG IN FORM VON HYDROPHOBEN PERLEN UND HERSTELLUNGSVERFAHREN
COSMETIC OR DERMOPHARMACEUTICAL COMPOSITION IN THE FORM OF HYDROPHOBIC BEADS AND METHODS FOR PREPARING SAME

(30) Priorité: 17.06.1998 FR 9807612
(43) Date de publication de la demande: 30.08.2000
(73) Titulaire: Ioualalen, Karim, 31650 Saint-Orens (FR); Raynal, Rosanne, 12430 Villefranche de Panat (FR)
(72) Inventeur: Ioualalen, Karim, 31650 Saint-Orens (FR); Raynal, Rosanne, 12430 Villefranche de Panat (FR)
(74) Mandataire: Santarelli
(86) Numéro de dépôt international: PCT/FR1999/001445
(87) Numéro de publication internationale: WO 1999/065448

(56) Documents cités:
- EP-A- 0 720 845
- WO-A-96/29056
- FR-A- 885 716
- FR-A- 2 486 800
- GB-A- 1 357 731
- US-A- 4 800 076
- V.D. VILIVALAM ET AL.: "development and evaluation of controlled-release diclofenac microspheres and tabletted microspheres" JOURNAL OF MICROENCAPSULATION, vol. 11, no. 4, 1 juillet 1994 (1994-07-01), pages 455-470, XP000451975 London /GB)

## Description

La présente invention a pour objet un nouveau type de perle biocompatible capable de contenir diverses substances, des composants à activité biologique, des composants cosmétiques ou pharmaceutiques, des pigments ou produits de charges.

L'utilisation d'actifs, ou de tous composés ou substances, pour des applications topiques à visées cosmétiques ou dermo-pharmaceutiques, nécessite l'incorporation à un support, dénommé composition pour les applications cosmétologie et support galénique pour les applications pharmaceutiques.

Les fonctions de ce support sont multiples :
- il doit permettre une application pratique au point d'administration. Dans le cas d'une application topique, à visée cosmétologique ou dermo-pharmaceutique, un fluide de comportement rhéologique adapté est la forme de support la plus souvent retenue.
- il doit permettre la dilution du principe actif ou des composés actifs pour obtenir la concentration souhaitée compatible avec l'effet recherché
- il doit apporter dans le cas des produits cosmétiques, un toucher le plus agréable possible dans ce cas les propriétés sensorielles du support sont primordiales

La plupart des compositions topiques, cosmétiques ou dermo-pharmaceutiques, sont des compositions aqueuses. On peut citer les gels, les lotions et les émulsions. Ces produits cosmétiques sont des démaquillants, des toniques, des compositions amincissantes, des compositions anti-solaires, après-solaires, des produits capillaires, des produits d'hygiène, des dentifrices et certains produits de maquillage. Ce sont aussi des compositions dermo-pharmaceutiques.

Ces compositions aqueuses sont aujourd'hui très utilisées en raison de leur forte teneur en eau qui procure lors de l'application une sensation de fraîcheur sur la peau et la muqueuse. Elles ne laissent pas de résidus, ne brillent pas et ne confèrent pas d'aspect ou de toucher gras à la peau.

Cependant ces compositions aqueuses, en particulier celles à phase aqueuse continue, présentent un certain nombre d'inconvénients rappelés dans le brevet FR 2 660554. Brièvement, on notera l'effet desséchant de ces compositions sur la peau, qui est mal supporté par les personnes à peaux sèche ou normale. La capacité de gels et émulsions à pouvoir incorporer certains composants comme les huiles essentielles ou les parfums sans être déstabiliser, reste faible.

Les compositions de type émulsion contenant une phase grasse, présentent une consistance très onctueuse. Elles sont très recherchées car elles sont facilement appliquées à l'aide des doigts à partir du conditionnement sous forme de pot ou de tube (Brevet FR 2734714). La grande stabilité de ces émulsions, est due à la présence de cires dans la phase grasse dont la température de fusion est généralement supérieure à 80°C. Leur préparation à une température supérieure à la température de fusion des cires, empêche l'utilisation de principes actifs thermosensibles.

En plus de l'effet desséchant observé, la phase aqueuse est un élément très favorable au développement de micro-organismes (Pharmacie galénique - Bonnes pratiques de fabrication des médicaments, A. Le Hir, Ed. Masson, page 88). Il est donc nécessaire d'incorporer à ces préparations, des agents limitant le développement microbien à large spectre. Ces agents, appelés conservateurs, peuvent présenter pour certains, une certaine toxicité vis à vis de la peau. Ainsi l'utilisation de certains conservateurs est aujourd'hui très réglementée voire interdite pour certains comme le diisobutyl-phénoxy-éthoxy-éthyl-diméthyl-benzylammonium (Législation et règlementation-Produits cosmétiques et produits d'hygiène corporelle - Les éditions du Journal officiel-1997-page 229).

Enfin l'eau présente dans ces compositions cosmétiques et dermocosmétiques, constitue un milieu très favorable aux réactions radicalaires et en particulier aux réactions d'oxydations.

Ainsi l'incorporation de molécules très sensibles au stress oxydatif comme la vitamine C ou certains acides gras ou certaines molécules polyinsaturées, est très difficile du fait de la très faible stabilité dans le temps.

Dans le cas des compositions associant une phase aqueuse et une phase lipidique, la présence indispensable de molécules amphiphiles à pouvoir tensioactif, permet la dispersion stable et le mélange des deux phases. Ces molécules peuvent présenter une certaine agressivité pour la peau (Les molécules de la beauté, de l'hygiène et de la protection pages 33-38, les tensioactifs, Pierre le Perchec, Ed Nathan). On cherche donc à réaliser des compositions anhydres dans lesquelles des principes actifs peuvent être incorporés à des températures inférieures à 80°C et dont la texture est suffisamment onctueuse pour permettre un étalement satisfaisant avec les doigts.

Les formulations anhydres destinées aux applications cosmétiques et dermo-pharmaceutiques sont connues depuis longtemps. Les plus anciennes sont constituées d'huiles animales, végétales, minérales ou de leur mélange. Ces formes huileuses ne sont plus utilisées en raison de leur texture grasse. Les compositions de maquillage à phase grasse, sont surtout utilisées pour leurs propriétés d'adhérence à l'épiderme, de protection, de formation de film imperméable à l'eau. Les produits anhydres de maquillage se présentent le plus souvent sous forme de solide compact, par exemple rouge à lèvre, ou sous forme de crème. (Peau-Soins cosmétiques et Préparations dermo-pharmaceutiques-R. Raoult, Ed. Porphyre page 106). Ces compositions, si elles sont intéressantes, restent néanmoins très grasses et épaisses. D'autre part, leur stabilité implique la présence de cires dont les températures de fusion généralement supérieures à 80°C empêche l'emploi de principes actifs thermosensibles.

Une amélioration est proposée dans le brevet français FR 2 734714, avec l'utilisation dans la composition d'une association entre autre d'argile organomodifiée, de particules polymériques, de silice pyrogènée, dispersées dans une phase grasse sans utilisation de cire. Cette composition sous forme de gel anhydre, même si elle reste grasse, est suffisamment onctueuse pour être étalée avec les doigts. Elle peut incorporer des molécules thermosensibles. Cependant les argiles utilisées, sont modifiées par des sels d'ammonium quaternaire gras pouvant être irritants pour la peau. Enfin la phase grasse comporte des huiles à caractère amphiphile, confèrant un aspect brillant à la peau.

Après différentes études, nous avons constaté que les inconvénients des compositions de l'art antérieur, à savoir :
- l'effet desséchant, la présence de tensioactifs, d'antioxydants, l'impossibilité d'utilisation de molécules sensibles au stress oxydatif pour les compositions aqueuses
- l'effet gras, l'impossibilité d'utilisation de molécules thermosensibles, l'effet brillant et la difficulté d'étalement pour les compositions anhydres
pouvaient être résolus par l'utilisation de perles de composition strictement anhydre et dépourvues complètement d'agents tensioactifs, définissant l'objet de l'invention.

De façon surprenante, nous avons découvert que l'association d'huiles, de cires minérales, de cires animales ou végétales, de talc et de silice, donne une composition solide, stable dans le temps, pouvant contenir des actifs, et qui présente une texture non grasse, fondante, filmogène, facile à étaler sans donner de résidus, ni d'aspect brillant.

Cependant cette composition n'est pas assez onctueuse pour pouvoir être prélevée facilement à l'aide des doigts à partir d'un conditionnement sous forme de pot ou de tube.

Nous avons découvert que des particules préparées à partir de cette composition, utilisées seules, adhèrent suffisaminent à l'épiderme pour être prélevées aisément avec les doigts et être appliquées en conservant les propriétés de la composition. Nous nommerons perles, les particules issues de la mise en forme de ces compositions. La composition apparaît comme étant dans un état dispersé, permettant des applications topiques cosmétiques et dermo-pharmaceutiques.

La présente invention, dans l'un de ses aspects, a donc pour objet une composition comprenant au moins une cire hydrophobe, une huile et du talc, caractérisée en ce que :
- elle se présente sous forme de mélange solide hydrophobe,
- elle ne contient pas d'eau, ni d'agents tensioactifs, ni d'agents émulsionnants, et
- elle se présente sous forme de particules sphériques hydrophobes.
La température de fusion finale doit être comprise entre 15°C et 70°C et de préférence entre 20°C et 45°C.

Les compositions conformes à l'invention, sont constituées essentiellement de cire ou de mélanges de cires minérales, d'huiles minérales non grasses, non amphiphiles, de talc et de silices. Elles peuvent contenir en outre des additifs huileux pâteux ou solides, des ingrédients actifs liposolubles ou hydrosolubles.

La composition contient généralement de 0,1 % à 40 % de cire de paraffine ou d'un mélange de cire de paraffine et de cires choisies préférentiellement parmi :
- la cire de Carnauba
- la cire de Candellila
- la cire d'Alfa
- l'ozokérite
- les cires végétales comme la cire d'olivier, la cire de riz, la cire de jojoba hydrogénée ou les cires absolues de fleurs
- les cires d'abeilles et cires d'abeilles modifiées

Il est possible d'utiliser d'autres matrices cireuses, mais le mélange obtenu doit être caractérisé par un point de fusion inférieur à 90°C, par l'absence de composés tensioactifs, par un comportement hydrophobe et une non mouillabilité par l'eau.

Dans le mélange de cires, la cire de paraffine représente de préférence au moins 40 % en poids par rapport au poids total du mélange de cires.

Outre les cires mentionnées ci-dessus, la composition selon l'invention contient généralement une huile minérale ou un mélange d'huiles minérales, préférentiellement une huile de paraffine ou un mélange d'huiles de paraffine et d'huile minérales ou d'huiles de synthèses choisies parmi :
- les huiles de silicones hydrophobes de viscosité comprise entre 5 et 9000 centistockes et plus particulièrement :

- les cyclométhicones
- les huiles organofluorées lipophiles
- le perhydrosqualène

Dans le mélange des huiles, l'huile de paraffine représente de préférence de 4 % à 90 % en poids par rapport au mélange. D'autres composés huileux comme l'alcool oléique, la lanoline, l'huile de tournesol, l'huile de palme, peuvent être utilisés, mais le mélange huileux obtenu doit être caractérisé par un comportement hydrophobe, une absence de miscibilité avec l'eau et un point de fusion compris entre 0°C et 45 °C.

Outre les huiles et les cires mentionnées ci-dessus, la composition selon l'invention contient du talc parfois additionné de silice. On choisi préférentiellement un talc de granulométrie fine, inférieure à 5 micromètres et une silice pyrogènée n'ayant pas subie de dérivation chimique. La composition contient généralement de 0,5 % à 10 % en poids de particules de talc et de silice.

Il est en outre possible pour ajuster la consistance, d'introduire dans la composition des argiles ou leurs dispersions huileuses, des gommes de silicones phénylées, des amidons, des structurants des corps gras.

Une forme particulièrement préférée de composition selon l'invention, contient en plus un polyoxyéthylène glycol, dont l'ajout permet d'améliorer les qualités de douceur et de fondant des compositions.

Les compositions selon l'invention sont obtenues par mélange à chauffage modéré. Plus précisément ces compositions sont obtenues par un procédé caractérisé par le fait que l'on mélange à la température de fusion de la cire, la cire et l'huile jusqu'à obtenir un mélange caractérisé par une température de fusion inférieure à la température de fusion de la cire. Le rapport initial entre la cire et l'huile peut être modulé pour que la température de fusion du mélange final, soit inférieure à la température de dégradation du composé à incorporer le plus sensible à la chaleur. Dans la pratique, le pourcentage en poids de cire hydrophobe dans le mélange est compris entre 1 et 40 % Le mélange final doit être solide à température ambiante et présenter préférentiellement un point de fusion de 30°C. Le mélange est ensuite refroidi sous agitation adaptée, à une température supérieure de 2°C ou 3°C à son point de fusion, pour permettre l'inclusion des actifs cosmétiques ou pharmaceutiques, des huiles de silicones, des polyoxyéthylènes-glycols, du talc, de la silice.

Les compositions obtenues sont ensuite mises en forme pour donner des particules sphériques hydrophobes appelées perles.

La présente invention dans l'un de ses aspects, a donc pour objet une composition anhydre, sans agents émulsionnants ou tensioactifs. La présente invention a également pour objet un procédé de préparation des perles à partir des compositions telles que définies ci-dessus.

Les principales techniques de préparation de particules sphéroïdes, sont essentiellement des techniques mécaniques. Par exemple, la granulation à chaud autour d'un noyau solide dans une turbine ou par un lit d'air fluidisé ou dans un mélangeur planétaire, par passage à travers une filière, par moulage, coulage ou injection à chaud ou sous pression dans des moules. Ces techniques ont toutes l'inconvénient de nécessiter un équipement important et ne permettent pas une modulation facile de la taille des particules. Les procédés sont longs et la composition est soumise à des stress thermique, mécanique et oxydatif dans le cas de la mise en forme sous pression à chaud.

La technique d'évaporation de solvant décrite dans le brevet FR 0 505 648, est basée sur la dispersion d'une phase organique contenant les produits constitutifs des particules et des agents dispersants, dans une phase aqueuse non miscible sous agitation. Cette étape est suivie de l'élimination sans agitation du solvant organique sous pression réduite, permettant la formation des particules. Cette méthode ne permet pas d'obtenir des particules de grande taille. De plus l'élimination complète du solvant est très difficile et enfin, les particules obtenues contiennent tout ou partie de l'agent dispersant.

Les techniques basées sur l'extrusion et/ou le broyage impliquent la formation de particules de surfaces irrégulières et soumettent la composition à des stress thermique, mécanique et oxydatif.

Des techniques moins agressives ou requérant peu d'échauffement, existent pour le traitement de substances sensibles à la température, à l'oxydation et au stress mécanique. Ce sont les méthodes dites de solidification par le froid.

Dans la description du brevet EP 0 438359, le mélange fondu contenant les actifs et les excipients, est dispersé sous forme de gouttelettes par passage à travers une buse, dans une tour où elles sont refroidies par un gaz froid à contre courant, puis récupérées. Si cette technique présente une nette amélioration par rapport aux techniques précédentes, elle reste complexe à mettre en oeuvre, l'étape de dispersion est difficile à maîtriser et la taille des particules n'est pas facilement modulable. On ne peut pas obtenir ou difficilement des particules dont la taille est supérieure à un millimètre. Enfin, cette technique ne permet pas d'obtenir de grands rendements.

Les techniques basées sur le refroidissement de la composition fondue, maintenue en mouvement dans un liquide non solvant comme l'eau, décrite dans les brevets FR 2 705232, FR 2 660 534, EP 0 045 008, sont appelées techniques par fusion chaude

Ces techniques décrites par Langer (E. Mathiowicz et R. Langer, Journal of Controlled Release, 5, 1987, pages 13-22), consistent à disperser la composition ou mélange, préalablement fondue, dans un liquide porté à la même température, avec lequel elle n'est pas miscible. Les gouttelettes obtenues, sont solidifiées par refroidissement du milieu de dispersion sous agitation. L'homme de l'art sait que l'une des grandes difficultés de ces procédés, est d'obtenir une bonne stabilisation de l'émulsion réalisée. En effet, si l'agitation n'est pas suffisante, les gouttelettes ont tendance à fusionner pour reconstituer les deux phases. L'émulsion doit être stabilisée sans variations de la taille des gouttelettes jusqu'à refroidissement sous la température de solidification. La taille des gouttelettes dépend de la vitesse d'agitation, de la viscosité du milieu de dispersion, et bien sûr de la présence d'agents tensioactifs ou émulsionnants. Dans les brevets FR 2 658829 et EP 0 045008, la dispersion est stabilisée par la présence d'agents amphiphiles et par le maintien d'une agitation importante. Si ces procédés sont intéressants, ils ne permettent pas d'obtenir des particules de tailles supérieures à 1000 micromètres et l'élimination des agents tensioactifs reste très difficile.

Dans la description du brevet FR 2 660554, le procédé ne fait pas appel à l'utilisation d'agent émulsionnant, car la composition lipidique contient des composés amphiphiles permettant de stabiliser la dispersion en phase aqueuse. De telles compositions ne permettent pas la préparation de particules supérieures à 1000 micromètres par ce procédé, et les particules ainsi préparées peuvent s'hydrater et absorber jusqu'à huit fois le poids d'eau. Ce procédé n'est donc pas compatible avec les compositions strictement hydrophobes.

Dans la description du brevet FR 2 705232, le mélange du principe actif à la composition à base de polymère hydrophobe, est réalisé lors de la dispersion dans une phase aqueuse à une température supérieure à la température de transition vitreuse. Ce procédé a l'inconvénient d'être long car la dispersion à chaud doit être maintenue jusqu'à la fin de l'encapsulation de composés actifs par les gouttelettes.

Selon l'invention, il est proposé un nouveau procédé pour la préparation de particules, à partir des compositions conformes à la présente invention, dans lesquelles les inconvénients des techniques décrites dans les procédés précédents, peuvent être évités.

En comparaison avec les procédés des brevets mentionnés ci-dessus, le procédé de la présente invention, est réalisé sans utiliser d'agent émulsionnant, ni solvant, ni particules préformées et il est beaucoup plus rapide. Les particules obtenues selon la présente invention, sont sphériques, de tailles comprises entre 1 et 10 000 micromètres. Selon une forme de réalisation préférée de l'invention, les perles ont une taille comprise entre 1000 et 10 000 micromètres et contiennent, dissous ou dispersés dans leur matrice, des composants cosmétiques, pharmaceutiques, biotechnologiques. Selon cette forme de réalisation, la composition lipidique des perles sert de support et de véhicule à des composants tels que des parfums, des huiles essentielles, des arômes, des pigments, des charges, des colorants, des enzymes et coenzymes et d'autres substances actives cosmétiques. La capacité de chargement des perles peut s'étendre de 0,02 % à 75 % par rapport au poids de perles.

L'homme de l'art sait que lorsqu'on effectue l'incorporation de ces composants dans les perles, il convient de choisir une composition lipidique appropriée de telle sorte que le procédé puisse être mis en oeuvre et que les perles soient solides à température ambiante avec une taille comprise de préférence entre 1000 et 10 000 micromètres.

Parmi les composants cosmétiques pouvant être incorporés dans les perles, on peut citer les vitamines ou provitamines A, B, C, D, E, PP et leurs esters, des hydratants, de la mélanine, les caroténoïdes, les substances anti-radicalaires, les hydroxyacides, les antiseptiques, les antiacnéïques, les antipelliculaires, les filtres U.V., les kératolytiques tels que l'acide salicylique et ses sels, les molécules agissant sur la pigmentation, sur l'inflammation, les agents émollients tels que les esters gras, les substituts ou constituants du sébum, les extraits biologiques, les colorants du cheveu, les antiperspirants, les molécules capables de piéger les odeurs et arômes, les molécules dites à effet amincissant. D'autres composants peuvent être incorporés dans les perles, comme les charges adoucissantes ou lubrifiantes pour la peau tels que le talc, le kaolin, le mica, les nanotitanes, les poudres de microbilles polymériques de polyamides ou au contraire des poudres destinées à l'abrasion à partir de particules de silices ou d'origine polymérique ou d'origine végétale.

Les perles peuvent contenir aussi des conservateurs, des antioxydants, des colorants et pigments ainsi que des agents matifiants comme par exemple le carbonate de magnésium, l'amidon, la poudre de zinc, l'oxyde de zinc, les microbilles siliconées Tospearl de Toshiba. On peut incorporer à ces perles d'autres microparticules ou microcapsules, des systèmes vecteurs ou matriciels contenant des principes actifs, des cellules et organites cellulaires. Enfin, ces perles peuvent contenir des composants pharmaceutiques destinés à traiter des pathologies cutanées ou mucosales ou capables de diffusion transdermique.

Dans cette description, le terme composant actif est utilisé pour désigner n'importe quelle substance thérapeutique active ou mélange pouvant être avantageusement administrés à l'homme ou aux autres animaux pour diagnostiquer, soigner, réduire, traiter ou prévenir la maladie. A titre d'exemples, on peut citer les antibiotiques, les hormones et dérivés, la nicotine, les antihistaminiques, les anti-inflammatoires stéroïdiens et non stéroïdiens, les agents antiallergiques, les anesthésiques locaux, les vasodilatateurs, les antiviraux, les anticorps et molécules agissant sur le système immunitaire. Cette liste exhaustive n'est en aucun cas limitative.

Dans le cas d'une administration par voie orale de ce type de particules, il convient de choisir une composition appropriée, compatible en terme de toxicité, de biocompatibilité, et de biodégradabilité avec l'absorption par voie orale. Dans ce cas les composants seront choisis parmi les composants déjà utilisés pour l'administration par voie orale et de telle sorte que les perles formées conservent leurs propriétés d'incorporation et de stabilisation des composants actifs.

Les perles selon l'invention peuvent contenir des pigments minéraux ou organiques y compris des pigments nacrés. Parmi les pigments organiques, on peut citer le noir de carbone, différents pigments organiques D et C Red, orange ou yellow codifiés dans le colour index. Parmi les colorants minéraux, on peut citer les dioxydes de titane, les oxydes de fer, rouge, brun, noir ou jaune, les oxydes de chrome, les outremers polysulfure d'aluminosilicate, le pyrophosphate de manganèse, le bleu de Prusse ou ferrocyanure ferrique et les bleus cyaniques. Les pigments sont présents dans les perles dans des concentrations allant de 0 à 30 % et préférentiellement de 1 % à 20 % en poids par rapport au poids de perle.

Les perles selon l'invention chargées en pigments, peuvent être utilisées comme bases de produit de maquillage tels que des fonds de teint, des embellisseurs de teint, des correcteurs de maquillage, des crèmes teintées, des fards à paupières, des fards à joues, des rouges à lèvres.

La présente invention dans l'un de ses aspects, a donc pour objet un procédé de préparation de perles contenant des pigments. Les perles contenant des pigments selon l'invention, peuvent être utilisées comme base de produits cosmétiques. Selon cette forme de réalisation, la taille des perles peut être inférieure à 10 micromètres. Dans ces conditions, les perles peuvent être utilisées pour l'incorporation de pigments destinés à l'élaboration de peintures, encres et teintures.

Selon l'invention, il est proposé un nouveau procédé pour la préparation de particules appelées perles, dans lequel les inconvénients des techniques décrites dans les procédés rappelés ci-dessus peuvent être évités.

En comparaison avec les techniques de fusion chaude, le procédé de la présente invention, ne fait pas intervenir d'agents émulsionnants ni de produits amphiphiles dans la composition, pour permettre une dispersion stable lors de la phase de solidification par refroidissement. Le mélange des différents composants et principes actifs constituant la composition à partir de laquelle seront obtenues les perles, est réalisé dans une première étape du procédé. Ce mélange est réalisé à chaud à 2°C ou 3 °C au dessus du point de fusion du composé présentant le point de fusion le plus élevé. L'homme de l'art sait qu'il est nécessaire d'appliquer un mode d'agitation approprié à la dispersion de tous les composants. Puis la formation des gouttelettes de la composition est réalisée en dispersant la composition dans un gel préalablement porté à la même température, avec lequel elle est non miscible, de viscosité suffisamment élevée pour figer la dispersion. Il est préférable d'injecter la composition au sein du gel, par exemple par un orifice situé à la base du réacteur. L'agitation qui doit être maintenue tout au long de l'injection a pour caractéristique de présenter une pale équipée d'une ancre, destinée à disperser la composition et une deuxième pale axiale équipée d'une hélice tripale destinée à former les gouttelettes de dispersion de taille souhaitée. Cette dernière étape est extrêmement rapide puisque les gouttelettes sont obtenues au fur et à mesure de l'injection de la composition. L'agitation n'a pas besoin d'être maintenue car les gouttelettes sont figées dans le gel. Dès la fin de l'injection, elles sont refroidies immédiatement sous la température de solidification puis lavées et récupérées sèches.

Ce procédé est donc rapide et ne nécessite pas d'étape d'agitation longue et délicate, il permet d'incorporer le principe actif à la composition dès la première phase de mélange des différents produits de la composition.

Parmi les agents gélifiants appropriés pour la formation des gels utilisés comme milieu de dispersion selon l'invention, on peut citer les polymères carboxyvinyliques tels que les carbopols neutralisés par la soude, les carraghénanes, les épaississants et gélifiants polysaccharidiques comme les xanthanes, les gommes de guar et de caroube, les alginates, les dérivés de cellulose, les pectines, l'agar.

Les gels utilisés dans le procédé de préparation des perles, ont une concentration généralement comprise entre 0,1 % et 50 % en poids par rapport au poids total de gel.

Les perles obtenues présentent une grande homogénéité de taille et peuvent être manipulées industriellement sans précaution particulière. Les exemples qui suivent ne sont pas limitatifs, ils servent seulement à illustrer l'invention.

### EXEMPLES

### Exemple 1 : Perles contenant des pigments

Exemple donné pour la fabrication de 100 g de perles contenant des pigments : Composition :
- Huile de paraffine 56 g
- Paraffine 15 g
- Cire d'abeille 2 g
- Huile de silicone 5 g
- Oxyde de fer jaune 3 g
- Oxyde de fer rouge 2 g
- Oxyde de fer noir 0,5 g
- Dioxyde de titane 6 g
- Talc 5 g
- Silice 2 g
- Filtre solaire Parsol 3 g
- Parfum 0,2 g
- Conservateur 0,3 g

### Mode opératoire :

Dans un récipient thermostaté, l'ensemble des substances de la composition est mélangé à chaud à 2°C ou 3°C au dessus du point de fusion du composé présentant le point de fusion le plus élevé. On ajoute en dernier les composants les plus fragiles. Dans le cas ou des composants ne sont pas miscibles dans la composition, la dispersion de ces composants dans la phase lipidique, est réalisée à l'aide d'une turbine à une vitesse de 1000 tours/min . Lorsque le mélange est homogène, il est ajouté à 600 ml de gel aqueux à 4 % de carbopol Ultrez 10, neutralisé à pH 6,5 avec de la soude, préalablement porté à la même température que le mélange et contenu dans un réacteur équipé d'un double système d'agitation à ancre et à hélice tripale. Pendant l'addition de la composition, la vitesse d'agitation de l'ancre est de 40 tours/min. et la vitesse d'agitation de l'hélice tripale est de 135 tours/min.. L'agitation est maintenue pendant 2 minutes après la fin de l'addition de la composition, puis stoppée. La dispersion est alors refroidie à 15°C. Les perles sont récupérées par tamisage puis lavées à l'eau distillée et récupérées. Les perles ainsi obtenues ont une taille de 3 mm et peuvent être utilisées comme fond de teint.

### Exemple 2 : Perles contenant des vitamines

Composition:
- Huile de paraffine 55 g
- Paraffine 16 g
- Huile de silicone 6 g
- Polyoxyéthylène glycol 400 6 g
- Talc 6 g
- Vitamine E 0,5 g
- Provitamine A 0,3 g
- Silice 4 g
- Dioxyde de titane 3 g
- Filtre solaire 3 g
- Conservateur 0,2 g

### Mode opératoire :

Le mode opératoire est identique à celui décrit dans l'exemple 1

Les perles obtenues ont une taille de 3,5 mm et peuvent être utilisées comme soin anti-âge.

### Exemple 3 : Perles chargées en huiles essentielles et huiles végétales

Composition :
- Huile de vaseline liquide 62 g
- Paraffine 16 g
- Cire d'abeille 1 g
- Huile de silicone 9 g
- Huile d'amande douce 2 g
- Essence de thym 0,5 g
- Essence de marjolaine 0,5 g
- Vitamine E 0,5 g
- Talc 5 g
- Kaolin 1 g
- Silice 1 g
- Parfum 0,2 g
- Conservateur 0,3 g

### Mode opératoire :

Le mode opératoire est identique à celui décrit dans l'exemple 1 Les perles obtenues ont une taille de 3 mm et peuvent être utilisées comme soin adoucissant et apaisant.

### Exemple 4 : Perles contenant des produits anti-solaires

Composition :
- Huile de paraffine 46 g
- Paraffine 22,8 g
- Huile de silicone 10 g
- Cire de Carnauba 1 g
- Lanoline 1 g
- Filtre anti-solaire Solécran 6 g
- Talc 5 g
- Silice 1 g
- Oxyde de zinc 2 g
- Dioxyde de titane 5 g
- Conservateur 0,2 g

### Mode opératoire:

Le mode opératoire est identique à celui décrit dans l'exemple 1 .

Les perles obtenues ont une taille de 4 mm et peuvent être utilisées comme soin de protection solaire.

### Exemple 5 : Perles contenant un pigment

Composition :
- Huile de paraffine 69 g
- Paraffine 15 g
- Noir de carbone 8 g
- Talc 8 g

### Mode opératoire :

Le mode opératoire reste identique à celui décrit dans l'exemple 1, excepté la concentration en carbopol Ultrez 10 dont la concentration est ramenée à 1,1 % dans cet exemple et la vitesse d'agitation de l'hélice tripale qui est ici de 400 tours/min.. Les perles chargées en pigments ainsi obtenues ont une taille de 25 micromètres et peuvent être utilisées dans l'industrie de l'encre, des teintures et des peintures.

### Exemple 6 : Perles chargées en erythromycine

Composition :
- Paraffine 64 g
- Huile de vaseline 18 g
- Talc 8 g
- P.E.G. 300 4 g
- Erythromycine base 4 g

### Mode opératoire :

Le mode opératoire reste identique à celui décrit dans l'exemple 1, excepté la concentration en carbopol Ultrez 10 dont la concentration est ramenée à 3,8. Les perles chargées en érythromycine ainsi obtenues, ont une taille de 2 mm et peuvent être utilisées pour les applications pharmaceutiques liées au traitement par antibiothérapie cutanée.

### Exemple 7 : Perles contenant des vitamines

Composition :
- Huile de palme 32 g
- Huile d'arachide 10 g
- Margarine 43,8 g
- Talc 10 g
- Vitamine E 0,5 g
- Vitamine C 2 g
- Conservateur 0,2 g

### Mode opératoire :

Le mode opératoire est identique à celui décrit dans l'exemple 1. Le gel de carbopol est remplacé par une solution visqueuse à 2% de gomme de caroube. Les perles obtenues présentent une taille de 1 mm et peuvent être utilisées comme complément alimentaire vitaminé.

### Exemple 8 : Perles contenant des cellules

Composition :
- Paraffine 49 g
- Huile de vaseline 6 g
- Huile de palme 20 g
- Saccharomycès Cerevisiae dessiqué 20 g
- Talc 5 g

### Mode opératoire :

Le mode opératoire est identique à celui décrit dans l'exemple 1. Les perles contenant des cellules présentent une taille de 1 mm.

### Exemple 9 : Préparation d'une formulation contenant des perles chargées en huiles essentielles pour obtenir un gel apaisant

On prépare un gel ayant la composition suivante :
- Carbopol Ultrez 10 0,15 g
- Glycérine 4 g
- Lanoline 4 g
- Conservateur 0,3 g
- Eau q.s.p. 100 g

A température ambiante, on incorpore à 60 g de ce gel, sous agitation, 40 g de perles chargées en huiles essentielles obtenues selon l'exemple 3.

## Revendications

1. Composition comprenant au moins une cire hydrophobe, une huile et du talc, **caractérisée en ce que** :
- elle se présente sous forme de mélange solide hydrophobe,
- elle ne contient pas d'eau, ni d'agents tensioactifs, ni d'agents émulsionnants, et
- elle se présente sous forme de particules sphériques hydrophobes.

2. Composition selon la revendication 1, **caractérisée en ce que** la température de fusion finale doit être comprise entre 15°C et 70°C.

3. Composition selon les revendications 1 ou 2, **caractérisée en ce que** l'huile utilisée est une huile choisie parmi les huiles minérales, les huiles synthétiques, les huiles silicones, les huiles fluorées, les huiles végétales ou leur mélange.

4. Composition selon l'une quelconque des revendications 1 à 3, **caractérisée en ce que** la cire hydrophobe est choisie parmi
- la cire de paraffine,
- la cire de Carnauba,
- la cire de Candellila,
- la cire d'Alfa,
- l' ozokérite,
- les cires végétales comme la cire d'olivier, la cire de riz, la cire de jojoba hydrogénée ou les cires absolues de fleurs,
- les cires d'abeilles et cires d'abeilles modifiées,
ou leur mélange.

5. Composition selon l'une des revendications 1 à 4, **caractérisée en ce que** l'huile est une huile non amphiphile.

6. Composition selon l'une des revendications 1 à 5, **caractérisée en ce que** l'huile est une huile de paraffine et la cire est une cire de paraffine.

7. Composition selon l'une quelconque des revendications 1 à 6, **caractérisée par** la présence d'huile de silicone comme adjuvant.

8. Procédé pour la préparation de particules sphériques hydrophobes selon l'une des revendications 1 à 7, consistant en une matrice lipidique hydrophobe de point de fusion compris entre 15 et 70°C, pouvant contenir au moins un composant actif, ledit procédé n'utilisant pas d'agents émulsionnants et comprenant les étapes suivantes :
- le mélange des différents composants et principes actifs est réalisé à chaud à 2°C ou 3°C au dessus du point de fusion du composé présentant le point de fusion le plus élevé ;
- la dispersion par injection sous agitation du mélange dans un gel préalablement porté à la même température que le mélange, avec lequel il est non miscible, de viscosité suffisamment élevée pour figer la dispersion des particules formées ;
- l'arrêt de l'agitation dès la fin de l'injection et refroidissement de la dispersion sous la température de solidification des particules ;
- la récupération et le lavage des particules.

9. Procédé selon la revendication 8, **caractérisé en ce que** l'addition du mélange est réalisée par injection sous agitation à la partie inférieure du récipient contenant le gel.

10. Procédé selon l'une des revendications 8 ou 9, **caractérisé en ce que** l'agitation du gel lors de l'injection, est obtenue à l'aide d'une pale équipée d'une ancre et d'une pale coaxiale équipée d'une hélice.

11. Procédé selon l'une des revendications 8 à 10, **caractérisé en ce que** le gel est préparé à partir d'agents gélifiants choisis parmi les polymères carboxyvinyliques tels que les carbopols, les carraghénanes, les épaississants et gélifiants polysaccharidiques comme les xanthanes, les gommes de guar et de caroube, les alginates, les dérivés de cellulose, les pectines, l'agar.

12. Procédé selon l'une des revendications 8 à 11, **caractérisé en ce que** le gel présente une concentration en agent gélifiant comprise entre 0,1 % et 50 % en poids par rapport au poids total de gel.

13. Particules sphériques hydrophobes, **caractérisées en ce qu'**elles sont préparées selon l'un des procédés des revendications 8 à 12.

14. Particules sphériques hydrophobes selon l'une des revendications 1 à 7
ou 13, **caractérisées en ce que** leur taille est comprise entre 1 et 10000 µm.

15. Particules sphériques hydrophobes selon l'une des revendications 1 à 7, 13 ou 14, **caractérisées en ce qu'**elles contiennent un ou plusieurs composants actifs cosmétiques
ou pharmaceutiques solubles ou non dans les perles.

16. Particules sphériques hydrophobes selon l'une des revendications 1 à 7 ou 13 à 15, **caractérisées en ce qu'**elles contiennent au moins un composant actif cosmétique ou pharmaceutique choisi parmi les vitamines ou provitamines A, B, C, D, E, PP et leurs esters, les hydratants, la mélanine, les caroténoïdes, les substances anti-radicalaires, les hydroxyacides, les antiseptiques, les antiacnéiques, les antipelliculaires, les filtres U.V., les kératolytiques tels que l'acide salicylique et ses sels, les molécules agissant sur la pigmentation, sur l'inflammation, les agents émollients tels que les esters gras, les substituts ou constituants du sébum, les extraits biologiques, les colorants du cheveu, les antiperspirants, les molécules capables de piéger les odeurs et arômes, les molécules dites amincissantes, ou d'autres composants choisis parmi les charges adoucissantes ou lubrifiantes pour la peau tels que le talc, le kaolin, le mica, les nanotitanes, les poudres de microbilles polymériques de polyamides ou au contraire les poudres destinées à l'abrasion à partir de particules de silices ou d'origine polymérique ou d'origine végétale, les conservateurs, les antioxydants, les colorants et pigments ainsi que des agents matifiants comme par exemple le carbonate de magnésium, l'amidon, la poudre de zinc, l'oxyde de zinc, les microbilles siliconées, des microparticules ou des microcapsules, des systèmes vecteurs ou matriciels contenant des principes actifs, des cellules et organites cellulaires, les antibiotiques, les hormones et dérivés, la nicotine, les antihistaminiques, les antiinflammatoires stéroïdiens et non stéroïdiens, les agents antiallergiques, les anesthésiques locaux, les vasodilalateurs, les antiviraux, les anticorps et molécules agissant sur le système immunitaire.

17. Particules sphériques hydrophobes selon l'une des revendications 1 à 7 ou 13 à 16, **caractérisées en ce qu'**elles contiennent un pigment utilisé pour la préparation des encres, des peintures et des teintures.

18. Particules sphériques hydrophobes selon l'une des revendications 1 à 7 ou 13 à 17, **caractérisées en ce qu'**elles contiennent des composants autorisés pour les applications agro-alimentaires.

19. Composition cosmétique ou dermo-pharmaceutique **caractérisée par le fait qu'**elle contient en suspension dans la phase continue, des particules sphériques hydrophobes selon l'une quelconque des revendications 1 à 7 ou 13 à 18.

## Claims

1. Composition comprising at least one hydrophobic wax, an oil and talc,
**characterized in that**:
- it is in the form of a solid hydrophobic mixture,
- it does not contain water, surface active agents or emulsifying agents,
- it is in the form of spherical hydrophobic particles.

2. Composition according to claim 1, **characterized in that** the final melting temperature must be comprised between 15°C and 70°C.

3. Composition according to claims 1 or 2, **characterized in that** the oil used is an oil chosen from the mineral oils, synthetic oils, silicone oils, fluorinated oils, vegetable oils or a mixture thereof.

4. Composition according to any one of claims 1 to 3, **characterized in that** the hydrophobic wax is chosen from
- Paraffin wax
- Camauba wax
- Candelilla wax,
- Esparto wax,
- Ozocerite
- Vegetable waxes such as olive wax, rice wax, hydrogenated jojoba wax or absolute flower waxes,
- Bees' wax and modified bees' wax,
or a mixture thereof.

5. Composition according to one of claims 1 to 4, **characterized in that** the oil is a non-amphiphilic oil.

6. Composition according to one of claims 1 to 5, **characterized in that** the oil is a paraffin oil and the wax is a paraffin wax.

7. Composition according to any one of claims 1 to 6, **characterized by** the presence of silicone oil as adjuvant.

8. Method for the preparation of spherical hydrophobic particles according to one of claims 1 to 7, consisting of a lipidic hydrophobic matrix with a melting point comprised between 15 and 70°C, which is able to contain at least one active constituent, said method not using emulsifying agents and comprising the following stages:
- hot mixing of the different constituents and active ingredients is carried out at 2°C or 3°C above the melting point of the compound having the highest melting point;
- dispersion by injection under stirring of the mixture in a gel previously heated to the same temperature as the mixture, with which it is non-miscible, with a sufficiently high viscosity to fix the dispersion of the particles formed;
- stopping the stirring at the end of the injection and cooling the dispersion below the temperature for solidifying the particles;
- recovery and washing of the particles.

9. Method according to claim 8, **characterized in that** the addition of the mixture is carried out by injection under stirring at the lower part of the container containing the gel.

10. Method according to one of claims 8 or 9, **characterized in that** the stirring of the gel during injection is obtained using a blade equipped with an anchor and a coaxial blade equipped with a propeller.

11. Method according to one of claims 8 to 10, **characterized in that** the gel is prepared using gelling agents chosen from the carboxyvinyl polymers such as carbopols, carrageenans, polysaccharide thickeners and gelling agents such as xanthans, guar and carob bean gums, alginates, cellulose derivatives, pectins, agar.

12. Method according to one of claims 8 to 11, **characterized in that** the gel has a concentration of gelling agent comprised between 0.1 % and 50 % by weight relative to the total weight of gel.

13. Spherical hydrophobic particles, **characterized in that** they are prepared according to one of the methods of claims 8 to 12.

14. Spherical hydrophobic particles according to one of claims 1 to 7 or 13, **characterized in that** their size is comprised between 1 and 10000 µm.

15. Spherical hydrophobic particles according to one of claims 1 to 7, 13 or 14, **characterized in that** they contain one or more cosmetic or pharmaceutical active constituents soluble or not in the pearls.

16. Spherical hydrophobic particles according to one of claims 1 to 7 or 13 to 15, **characterized in that** they contain at least one cosmetic or pharmaceutical active constituent chosen from the vitamins or provitamins A, B, C, D, E, PP and their esters, moisturizers, melanin, carotenoids, anti-radical substances, hydroxyacids, antiseptics, anti-acne agents, anti-dandruff agents, U.V. filters, keratolytics such as salicylic acid and its salts, molecules acting on pigmentation and on inflammation, emollients such as fatty esters, sebum substitutes or constituents, biological extracts, hair dyes, antiperspirants, molecules capable of trapping odours and aromas, so-called slimming molecules, or other constituents chosen from softening or lubricating agents for the skin such as talc, kaolin, mica, nano titaniums, polyamide polymer microbead powders or, on the contrary, powders intended for abrasion using particles of silica or originating from polymers or plants, preservatives, antioxidants, colorants and pigments as well as matting agents such as for example magnesium carbonate, starch, zinc powder, zinc oxide, silicon-containing microbeads, microparticles or microcapsules, vector or matrix systems containing active ingredients, cells and cellular organites, antibiotics, hormones and derivatives, nicotine, antihistaminics, steroidal and non-steroidal anti-inflammatory drugs, antiallergic agents, local anesthetics, vasodilators, antivirals, antibodies and molecules acting on the immune system.

17. Spherical hydrophobic particles according to one of claims 1 to 7 or 13 or 16, **characterized in that** they contain a pigment used for the preparation of inks, paints and dyes.

18. Spherical hydrophobic particles according to one of claims 1 to 7 or 13 to 17, **characterized in that** they contain constituents authorized for applications in the agri-food industry.

19. Cosmetic or dermo-pharmaceutical composition **characterized in that** it contains spherical hydrophobic particles according to any one of claims 1 to 7 or 13 to 18, in suspension in the continuous phase.

## Patentansprüche

1. Zusammensetzung, die wenigstens ein hydrophobes Wachs, ein Öl und Talk umfaßt, **dadurch gekennzeichnet, daß**
- sie in Form: eines festen hydrophoben Gemisches vorliegt;
- sie weder Wasser noch grenzflächenaktive Stoffe noch Emulgatoren enthält;
- sie in Form von sphärischen hydrophoben Partikeln vorliegt.

2. Zusammensetzung nach Anspruch 1, **dadurch gekennzeichnet, daß** die finale Schmelztemperatur zwischen 15°C und 70°C liegen muß.

3. Zusammensetzung nach Anspruch 1 oder 2, **dadurch gekennzeichnet, daß** das verwendete Öl ein Öl ist, das unter Mineralölen, synthetischen Ölen, Siliconölen, fluorierten Ölen, Pflanzenölen oder deren Gemisch ausgewählt ist.

4. Zusammensetzung nach einem der Ansprüche 1 bis 3, **dadurch gekennzeichnet, daß** das hydrophobe Harz ausgewählt ist aus
- Paraffinwachs,
- Carnaubawachs,
- Candellilawachs,
- Alfawachs,
- Ozokerit,
- Pflanzenwachsen, wie z.B. Ölbaumwachs, Reiswachs, hydriertes Jojobaöl oder reine Blumenwachse,
- Bienenwachsen und modifizierten Bienenwachsen, oder deren Gemisch.

5. Zusammensetzung nach einem der Ansprüche 1 bis 4,
**dadurch gekennzeichnet daß** das Öl ein nicht-amphiphiles Öl ist.

6. Zusammensetzung nach einem der Ansprüche 1 bis 5, **dadurch gekennzeichnet daß** das Öl ein Paraffinöl ist und das Wachs ein Paraffinwachs ist.

7. Zusammensetzung nach einem der Ansprüche 1 bis 6, **gekennzeichnet durch** das Vorliegen von Siliconöl als Adjuvans.

8. Verfahren zur Herstellung von sphärischen hydrophoben Partikeln nach einem der Ansprüche 1 bis 7, die aus einer hydrophoben Lipidmatrix mit einem Schmelzpunkt zwischen 15 und 70°C bestehen, wenigstens einen aktiven Bestandteil enthalten können, wobei das Verfahren keine Emulgatoren verwendet und die folgenden Stufen umfaßt:
- Mischen der verschiedenen Bestandteile und aktiven Prinzipien wird in der Wärme bei 2°C oder 3°C unterhalb des Schmelzpunkts der Verbindung, die den höchsten Schmelzpunkt aufweist, durchgeführt;
- Dispergieren des Gemisches durch Injektion unter Rühren in ein vorher auf dieselbe Temperatur wie das Gemisch gebrachtes Gel, mit dem es nicht mischbar ist, das ein ausreichend hohe Viskosität hat, um die Dispersion der gebildeten Partikel erstarren zu lassen;
- Anhalten des Rührens am Ende der Injektion und Abkühlen der Dispersion unter die Verfestigungstemperatur der Partikel;
- Isolierung und Waschen der Partikel.

9. Verfahren nach Anspruch 8, **dadurch gekennzeichnet, daß** der Zusatz des Gemisches durch Injektion unter Rühren zu dem unteren Teil des Behälters, der das Gel enthält, durchgeführt wird.

10. Verfahren nach einem der Ansprüche 8 oder 9, **dadurch gekennzeichnet, daß** das Rühren des Gels während der Injektion mit Hilfe eines Blatts, ausgestattet mit einem Anker, und eines coaxialen Blatts, ausgestattet mit einem Propeller, erreicht wird.

11. Verfahren nach einem der Ansprüche 8 bis 10, **dadurch gekennzeichnet, daß** das Gel ausgehend von Geliermitteln hergestellt wird, welche unter Carboxyvinyl-Polymeren wie zum Beispiel Carbopolen, Carraghenanen, polysaccharidischen Verdickungsmitteln und Geliermitteln wie zum Beispiel Xanthanen, Guargummi und Johannisbrotgummi, Alginaten, Cellulosee-Derivaten, Pektinen, Agar ausgewählt sind.

12. Verfahren nach einem der Ansprüche 8 bis 11, **dadurch gekennzeichnet, daß** das Gel eine Konzentration an Geliermittel zwischen 0,1 Gew.% und 50 Gew.%, bezogen auf das Gesamtgewicht des Gels, aufweist.

13. Sphärische hydrophobe Partikel, **dadurch gekennzeichnet, daß** sie nach einem der Verfahren nach den Ansprüchen 8 bis 12 hergestellt sind.

14. Sphärische hydrophobe Partikel nach einem der Ansprüche 1 bis 7 oder 13, **dadurch gekennzeichnet, daß** ihre Größe zwischen 1 und 10 000 µm liegt.

15. Sphärische hydrophobe Partikel nach einem der Ansprüche 1 bis 7, 13 oder 14, **dadurch gekennzeichnet daß** sie einen kosmetisch oder pharmazeutisch aktiven Bestandteil, der in den Perlen löslich ist oder nicht oder mehrere kosmetisch oder pharmazeutisch aktive Bestandteile, die in den Perlen löslich sind oder nicht, enthalten.

16. Sphärische hydrophobe Partikel nach einem der Ansprüche 1 bis 7 oder 13 bis 15, **dadurch gekennzeichnet, daß** sie wenigstens einen kosmetisch oder pharmazeutisch wirksamen Bestandteil enthalten, ausgewählt aus den Vitaminen oder Provitaminen A, B, C, D, E, PP und ihren Estern, Feuchtigkeit spendenden Mitteln, Melanin, Carotinoiden, Antiradikalsubstanzen, Hydroxysäuren, Antiseptika, Antiaknemitteln, Antischuppenmitteln, UV-Filtern, Keratolytika wie zum Beispiel Salicylsäure und ihre Salze, Molekülen, die auf die Pigmentierung, auf Entzündung wirken, weichmachenden Mitteln wie Fettsäureester, Ersatzstoffen oder Bestandteilen des Talgs, biologischen Extrakten, Haarfärbemitteln, Antiperspirantien, Molekülen, die fähig sind, Düfte und Aromen zu verleihen, sogenannten Schlankheitsmitteln, oder andere Bestandteile enthalten, ausgewählt aus weichmachende oder glättende Füllstoffe für die Haut wie zum Beispiel Talk, Kaolin, Glimmer, Nanotitane, Pulver aus polymeren Mikrokügelchen aus Polyamiden oder im Gegensatz dazu Pulver, die zum Abschleifen bestimmt sind, aus Siliciumdioxidpartikeln oder polymeren Ursprungs oder pflanzlichen Ursprungs, Konservierungsstoffe, Antioxidantien, Färbemittel und Pigmente sowie mattierende Mittel wie zum Beispiel Magnesiumcarbonat, Stärke, Zinkpulver, Zinkoxid, siliconisierte Mikrokügelchen, Mikropartikel oder Mikrokapseln, Vektorsysteme oder Matrizes, die aktive Prinzipien enthalten, Zellen oder Zellorganellen, Antibiotika, Hormone und Derivate davon, Nikotin, Antihistaminika, steroidale und nicht-steroidale Mittel gegen Entzündungen, Antiallergika, Lokalanästhetika, Vasodilatatoren, antivirale Mittel, Antikörper und Moleküle, die auf das Immunsystem wirken.

17. Sphärische hydrophobe Partikel nach einem der Ansprüche 1 bis 7 oder 13 bis 16, **dadurch gekennzeichnet daß** sie ein Pigment enthalten, das zur Herstellung von Tinten, Anstrichmitteln und Färbemitteln bzw. Tinkturen verwendet wird.

18. Sphärische hydrophobe Partikel nach einem der Ansprüche 1 bis 7 oder 13 bis 17, **dadurch gekennzeichnet, daß** sie Bestandteile enthalten, die für agroalimentäre Anwendungen zugelassen sind.

19. Kosmetische oder dermopharmazeutische Zusammensetzung, **dadurch gekennzeichnet, daß** sie in Suspension in einer kontinuierlichen Phase sphärische hydrophobe Partikel nach einem der Ansprüche 1 bis 7 oder 13 bis 18 enthält.
